Europäisches Patentamt

European Patent Office

Office européen des brevets

(11) Veröffentlichungsnummer: **0 315 013**
**A2**

(12) **EUROPÄISCHE PATENTANMELDUNG**

(21) Anmeldenummer: 88117685.3

(51) Int. Cl.⁴: **A41B 13/02 , A61F 13/18**

(22) Anmeldetag: 24.10.88

(30) Priorität: 02.11.87 DE 3737145
07.12.87 DE 3741405

(43) Veröffentlichungstag der Anmeldung:
**10.05.89 Patentblatt 89/19**

(84) Benannte Vertragsstaaten:
**AT BE CH DE ES FR GB GR IT LI LU NL SE**

(71) Anmelder: **Claassen, Henning J.**
**Industriegebiet Hafen**
**D-2120 Lüneburg(DE)**

(72) Erfinder: **Claassen, Henning J.**
**Industriegebiet Hafen**
**D-2120 Lüneburg(DE)**

(74) Vertreter: **Marx, Lothar, Dr. et al**
**Patentanwälte Schwabe, Sandmair, Marx**
**Stuntzstrasse 16 Postfach 86 02 45**
**D-8000 München 80(DE)**

(54) **Hygiene-Artikel und Verfahren zur Herstellung eines Hygieneartikels.**

(57) Bei einer Hygiene-Artikel mit einer Innenlage aus einem feuchtigkeitsdurchlässigen Material, einer Polsterlage aus einem Feuchtigkeit aufnehmenden, saugfähigen material und einer Außenlage besteht die feuchtigkeitsundurchlässige Außenlage aus einem Vliesstoff, der auf einer Seite mit einer dünnen Schicht aus einem thermoplastischen, hochpolymeren Werkstoff, insbesondere einem Hotmelt auf der Basis von Polyethylen, EVA oder ATP, versehen ist.

Zur Herstellung eines solchen Hygiene-Artikels werden gleichzeitig die Innenlage aus dem feuchtigkeitsdurchlässigen Material und die Vliesstoff-Bahn, die auf einer Seite mit dem thermoplastischen, hochpolymeren Werkstoff beschichtet ist, aud die zentrale Polsterlage aufkaschiert.

FIG. 1

## Hygiene-Artikel und Verfahren zur Herstellung eines Hygiene-Artikels

Die Erfindung betrifft einen Hygiene-Artikel der im Oberbegriff des Anspruchs 1 angegebenen Gattung sowie ein Verfahren zur Herstellung eines Hygiene-Artikels der im Oberbegriff des Anspruchs 9 angegebenen Gattung.

Unter dem Begriff "Hygiene-Artikel" werden hier insbesondere Frauenhygienische Artikel, also Damenbinden, Slipeinlagen, Monatshöschen und Still-Einlagen, aber auch Windeln für Babies oder Kranke sowie Krankenunterlagen verstanden. Im folgenden sollen die dabei auftretenden Probleme und ihre Lösung anhand von Baby-Windeln erläutert werden.

Übliche Baby-Windeln (z.B. DE-PS 24 54 590) weisen ein Feuchtigkeit aufnehmendes, also "saugfähiges", relativ dickes Polstermaterial aus Zellstoffflocken oder Vlies auf, das auf der Innenseite mit einer feuchtigkeitsdurchlässigen Innenlage, im allgemeinen aus einem Vliesstoff, und auf der Außenseite mit einer feuchtigkeitsundurchlässigen Außenlage, im allgemeinen aus einer Polyethylen-Folie, versehen ist. In Draufsicht hat eine solche Windel Doppel-T-Form, wobei die verjüngen, mittleren Bereiche im allgemeinen mit einem "Gummizug" versehen sind, der aus elastischen Bändern oder Fäden besteht und die Windel an die Beine anlegt, also Abdichtungs-Funktion erfüllt.

Zur Verbindung der beiden Enden der Windel miteinander und damit zur Befestigung der Windel am Körper sind im allgemeinen Befestigungsvorrichtungen vorgesehen, insbesondere Klebebänder.

Die Klebeflächen dieser Klebebänder werden auf die Außenfläche der Außenlage der Windel gedrückt; die üblichen Polyethylen-Folien halten den dabei auftretenden Beanspruchungen nicht stand, so daß ein zusätzliches Verstärkungsband verwendet werden muß, das an diesen Befestigungsstellen auf die Polyethylen-Außenlage aufgebracht wird. Außerdem ist die als Feuchtigkeitssperre dienende Außenlage, also Polyethylen-Folie, luftundurchlässig und damit beim Tragen unangenehm ist.

Der Erfindung liegt deshalb nach einem ersten Aspekt die Aufgabe zugrunde, einen Hygiene-Artikel der angegebenen Gattung zu schaffen, bei dem der oben erwähnte Nachteil nicht auftritt. Insbesondere soll ein Hygiene-Artikel vorgeschlagen werden, der beim Tragen angenehm ist.

Dies wird erfindungsgemäß durch die im kennzeichnenden Teil des Anspruchs 1 angegebenen Merkmale erreicht.

Zweckmäßige Ausführungsformen werden durch die Merkmale der Unteransprüche 2 bis 8 definiert.

Die mit der Erfindung erzielten Vorteile beruhen darauf, daß ein mit einer dünnen Schicht eines thermoplastischen, hochpolymeren Werkstoffes versehener Vliesstoff eine ausreichende mechanische Festigkeit hat, also z. B. kein zusätzliches Verstärkungsband für die Verbindung mit einer Befestigungsvorrichtung, im allgemeinen einem Klebeband, erforderlich ist. Auch an den übrigen Stellen hat diese Außenlage eine ausreichende Festigkeit und Widerstandsfähigkeit in Verbindung mit der angestrebten, hohen Undurchlässigkeit für Feuchtigkeit.

Ist die aufgebrachte Schicht aus dem thermoplastischen, hochpolymeren Werkstoff ausreichend dünn, so bleibt die Außenlage zwar feuchtigkeitsundurchlässig, wird jedoch gleichzeitig gas-, insbesondere luftdurchlässig; man kann sie als "atmungsaktiv" bezeichnen, so daß der freie Luftaustausch weitgehend möglich ist, wie es bei Außenlagen aus Polyethylen-Folien nicht erreicht werden kann.

Die Vliesbahn sollte aus einem hochpolymeren Fasermaterial, insbesondere Polypropylen-, Polyamid- oder Polyester-Fasern hergestellt werden, im wesentlichen also aus den gleichen Materialien wie sie für die Innenlage verwendet werden.

Als Beschichtungsmaterial kommen bevorzugt Hotmelts, insbesondere auf der Basis von Polyethylen, EVA oder ATP in Frage.

Obwohl im Prinzip die Möglichkeit besteht, die Beschichtung aus dem thermoplastischen, hochpolymeren Werkstoff auf die Außenfläche der Vliesstoff-Außenlage aufzubringen, wird nach einer bevorzugten Ausführungsform die dem Polstermaterial zugewandte Innenfläche des Vliesstoffes mit dem thermoplastischen, hochpolymeren Werkstoff versehen. Dadurch bildet die unbeschichtete Oberfläche der Vliesstoff-Außenlage die Außenfläche des Hygiene-Artikels, so daß sich ein "textiler Griff" ergibt, der sich positiv von dem "Plastik-Griff" der bisher verwendeten Polyethylen-Folien abhebt.

Die Schicht aus dem thermoplastischen, hochpolymeren Werkstoff wird nach einer bevorzugten Ausführungsform mit einer extrem geringen Flächendichte von 15 bis 30 g/m² auf den Vliesstoff aufgebracht, da durch Versuche festgestellt werden konnte, daß diese Flächendichte einmal die gewünschte "Feuchtigkeitssperre" bewirkt und trotzdem noch den freien Luftdurchtritt ermöglicht.

Die verwendete Flächendichte wird auf die jeweilige Vlies-Struktur und damit Durchlässigkeit abgestellt.

Nach einem weiteren Aspekt liegt der Erfindung die Aufgabe zugrunde, ein Verfahren zur Herstellung eines Hygiene-Artikels der im Oberbegriff des Anspruchs 9 angegebenen Gattung zu schaffen, das sich konstruktiv einfach, also mit geringem baulichen Aufwand, realisieren läßt.

Dies wird erfindungsgemäß durch die im kennzeichnenden Teil des Anspruchs 9 angegebenen Merkmale erreicht.

Zweckmäßige Ausgestaltungen dieses Verfahrens werden durch die Merkmale der Unteransprüche 10 bis 16 definiert.

Der besondere Vorteil dieses Verfahrens liegt in dem Aufkaschieren eines als Außenlage dienenden Vliesstoffes, der mit einem thermoplastischen, hochpolymeren Werkstoff beschichtet ist, direkt auf die Polsterschicht. Gleichzeitig werden die in aller Regel erforderlichen elastischen Bänder bzw. Fäden zwischen der Innen-Lage und der Außen-Lage einkaschiert, so daß sich ein einfach zu realisierender Verfahrensablauf ergibt.

Obwohl im Prinzip auch die Möglichkeit besteht, mit vorbeschichteten Vliesstoffen zu arbeiten, erfolgt nach einer bevorzugten Ausführungsform auch die Beschichtung des Vliesstoffes für die Außenlage "on-line", also unmittelbar vor dem Aufkaschieren auf das Polstermaterial, wodurch die sonst umständliche Handhabung des beschichteten Vliesstoffes entfällt. Zu diesem Zweck wird der mit dem bevorzugten thermoplastischen, hochpolymeren Material, nämlich einem Hotmelt auf der Basis von Polyethylen, EVA oder ATP, beschichtete Vliesstoff an einer Heizwalze vorbeigeführt, so daß der erwärmte und damit augeschmolzene Hotmelt mit dem Vliesstoff verbunden wird.

Die Aufbringung des thermoplastischen, hochpolymeren Werkstoffes erfolgt mittels einer Flächendüse oder einer Auftragswalze auf die Seite des Vliesstoffes, die beim Aufkaschieren dem Polstermaterial zugewandt wird, d.h. die Beschichtung befindet sich auf der Innenseite der Außenlage, so daß die Außenseite der Außenlage den erwähnten "textilen Griff" hat.

Die Erfindung wird im folgenden anhand von Ausführungsbeispielen unter Bezugnahme auf die beiliegenden, schematischen Zeichnungen näher erläutert.

Es zeigen

Fig. 1 eine Draufsicht auf eine Windel mit teilweise abgezogener Innenlage,

Fig. 2 eine perspektivische Ansicht einer Vorrichtung zur Herstellung dieser Windel, und

Fig. 3 eine perspektivische Ansicht einer Modifikation der Vorrichtung nach Fig. 2.

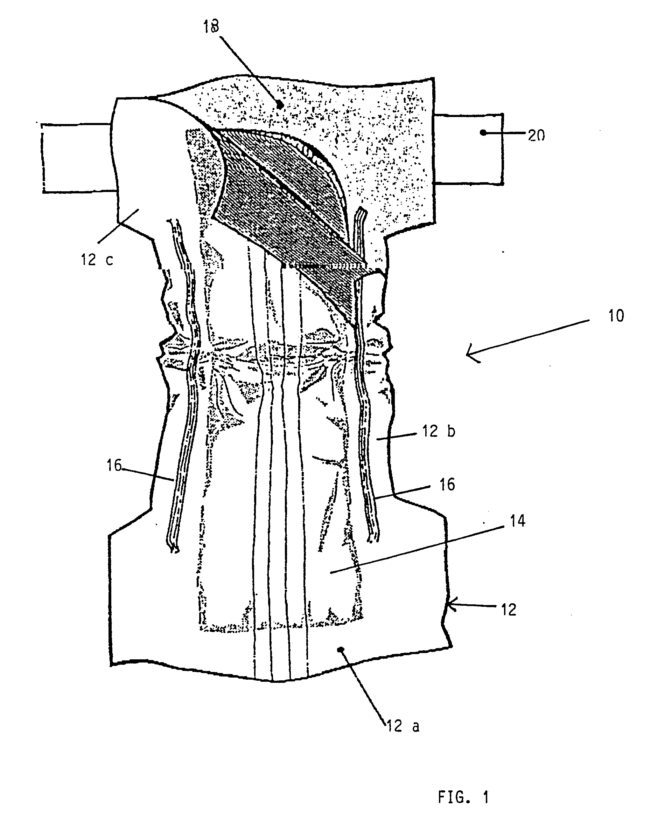

Die aus Figur 1 ersichtliche, allgemein durch das Bezugszeichen 10 angedeutete Windel weist eine Innenlage 12 aus einem feuchtigkeitsdurchlässigen Vliesstoff auf; diese Innenlage hat die übliche Doppel-T-Form, enthält also zwei relativ breite Endbereiche 12a, 12c und einen schmaleren Beinbereich 12b; sie besteht aus Polypropylen, Polyamid- oder Polyester-Fasern.

Ein relativ breiter Streifen aus einem feuchtigkeitaufnehmenden, saugfähigen Polstermaterial 14 auf der Basis von Zellstoff in Form von Flokken oder Vliesen erstreckt sich auf der Innenlage 12 von dem breiten Bereich 12a über den schmalen Bereich 12b in den anderen breiten Bereich 12c.

Auf der Außenseite der Windel 10 ist das Polstermaterial 14 durch eine atmungsaktive Außenlage 18 bedeckt, deren Form der Form der Innenlage 12 entspricht, also ebenfalls in Doppel-T-Form ausgebildet ist. Diese Außen-lage 18 besteht aus einem Vliesstoff aus einem textilen, hochpolymeren Material, insbesondere Polypropylen-, Polyamid- oder Polyester-Fasern, die entsprechend der Innenlage 12 mit den üblichen Technologien zu einem Vliesstoff verarbeitet worden sind.

Die Innenseite des Vliesstoffes der Außenlage 18, also die dem Polstermaterial 14 zugewandte Seite, ist mit einer sehr dünnen Schicht eines thermoplastischen, hochpolymeren Werkstoffes, insbesondere eines Hotmelt auf der Basis von Polyethylen, EVA oder ATP,versehen.

Die Außenseite der Außenlage 18 behält also ihren textilen Character, während die Innenseite durch die Verwendung des thermoplastischen, hochpolymeren Werkstoffes feuchtigkeitsundurchlässig ist, also keine Feuchtigkeit nach außen dringen kann. Die Dicke der Beschichtung wird so auf den Vliesstoff abgestellt, daß sich diese Wirkung ergibt.

Die dünne Schicht aus dem thermoplastischen, hochpolymeren Werkstoff, insbesondere einem Hotmelt, dient also als Feuchtigkeits-Sperre, ist jedoch gleichzeitig luftdurchlässig, so daß eine "atmungsaktive" Beschichtung entsteht.

Die Außenlage 18 hat eine so hohe Festigkeit und Widerstandsfähigkeit, daß ein zur Verbindung der beiden Enden der Windel 10 miteinander dienendes Klebeband 20 , das auf einer Seite eines T-Schenkels an der Außenfläche der Windel 10 angebracht ist, problemlos mit der gegenüberliegenden Außenfläche verklebt werden kann.

Es ist also keine zusätzliche Verstärkungslage an der Außenfläche der Windel 10 mehr erforderlich.

Im Beinbereich, also etwa im schmalen Bereich 12 b der Innenlage 12, sind in die Windel 10 neben dem Polstermaterial 14 Gummizüge eingearbeitet, die durch elastische Bänder oder Fäden 16 gebildet werden. In diesem Bereich legt sich also die Windel 10 an die Beine und die Leiste des Babys an, so daß sich eine gute Abdichtung ergibt.

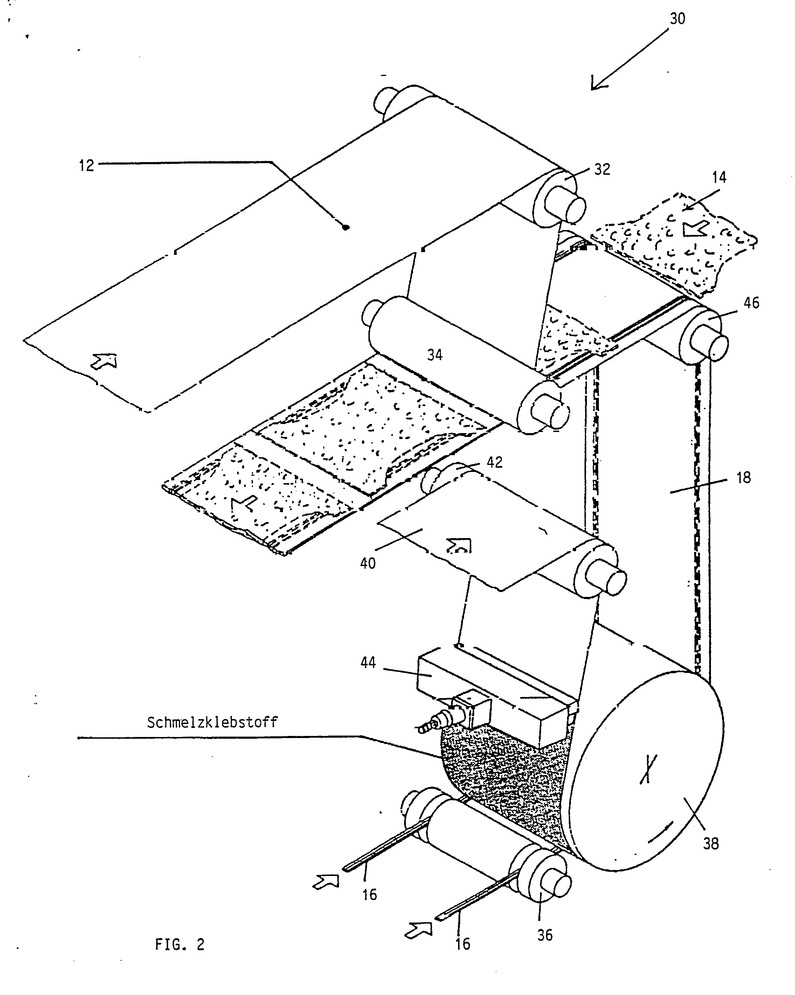

Figur 2 zeigt eine bevorzugte Ausführungsform einer Vorrichtung zur Realisierung eines Verfahrens zur Herstellung einer solchen windel. Dabei wird zunächst das Basismaterial, nämlich das Polstermaterial 14, in Richtung des Pfeils zu- und zwischen zwei Walzen 46, 34 hindurchgeführt. Bei diesem Durchlauf wird das Polstermaterial 14 von oben her mit dem Vliesstoff 12 für die Innenlage und von unten her mit dem beschichteten Vliesstoff 18 für die Außenlage kaschiert.

Zu diesem Zweck wird der Vliesstoff 12 für die Innenlage in Richtung des Pfeils von einem Vorrat (nicht dargestellt) zugeführt, um eine Walze 32 nach unten abgelenkt und dann mittels der Walze 34 auf die entsprechende Oberfläche des Polstermaterials 14 aufkaschiert.

In ähnlicher Weise wird von unten her der Vliesstoff 40 für die Außenlage 18 von einem Vorrat (nicht dargestellt) in Richtung des Pfeils zugeführt, um eine Walze 42 nach unten umgelenkt und dann an einer Flächendüse 44 vorbeigeführt, die auf die gesamte Breite des Vliesstoffes 40 eine dünne Schicht aus einem Hotmelt aufbringt. Es ist wesentlich, abgestimmt auf den Vliesstoff, mit möglichst geringen Flächengewichten zu arbeiten; gute Ergebnisse werden mit einem Flächengewicht von 23 g/m² erhalten.

Der Vliesstoff 40, der auf seiner gemäß der Darstellung in Figur 2 linken Seite mit dem Hotmelt versehen ist, wird nun um eine relativ große Heizwalze 38 geführt, die sich in Richtung des Pfeils dreht und den Hotmelt erwärmt. Gleichzeitig werden die beiden Gummizüge 16, die als geschlitzte Gummibänder dargestellt sind, in Richtung der Pfeile zugeführt und durch zwei Ringnuten in einer Walze 36 ausgerichtet, so daß sie in genau definierten Lagen um die Heizwalze 38 verlaufen und dabei in Anlage an die geschmolzene Hotmelt-Schicht auf dem Vliesstoff 40 gebracht werden. Die fertige Außenlage 18 mit der Hotmelt-Schicht und den integrierten, geschlitzten Gummibändern 16 wird dann von der Walze 38 nach oben transportiert und mittels der Walze 36 so auf die Unterseite des Polstermaterials (gemäß der Darstellung in Figur 2) kaschiert, daß die Hotmelt-Schicht der Außenlage 18 dem Polstermaterial 14 zugewandt ist. Auf diese Weise werden gleichzeitig die beiden Gummibänder 16 neben dem Polstermaterial 14 zwischen die beiden Lagen 12 und 18 einkaschiert.

Als Alternative zu der dargestellten Ausführungsform besteht auch die Möglichkeit, mit Hotmelt vorbeschichtete Vliesstoffe 14 zu werden, also Vlies-Bahnen, die in beschichtetem Zustand angeliefert und eingesetzt werden.

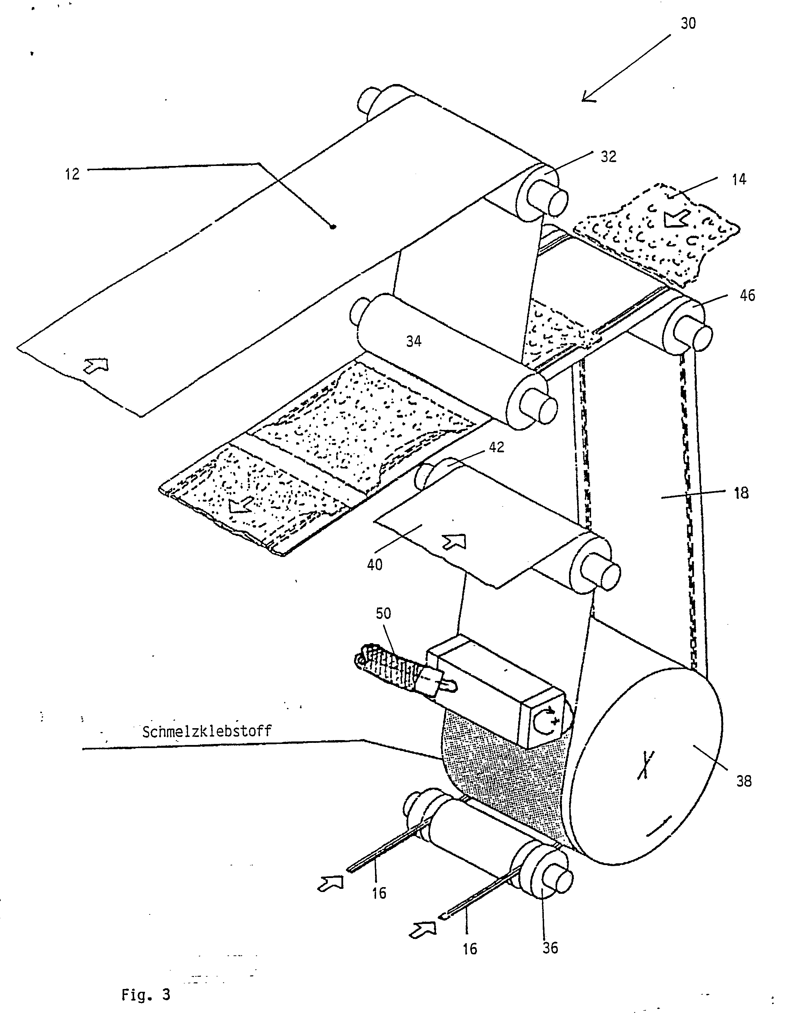

Fig. 3 zeigt eine Modifikation der Vorrichtung nach Fig. 2, bei der die Flächendüse 44 durch eine Auftragwalze 50 ersetzt ist. Versuche haben ergeben, daß mit einer solchen Auftragwalze extrem geringe Flächendichte von bis zu 15 g/m² erreicht werden können, ohne daß angestrebte geschlossene Hotmelt-Film auf dem Vliesstoff 40 reißt und damit seine einwandfreie Funktion als Feuchtigkeitssperre gefährdet wird.

## Ansprüche

1. Hygiene-Artikel
   a) mit einer Innenlage aus einem feuchtigkeitsdurchlässigen Material,
   b) mit einer Polsterlage aus einem Feuchtigkeit aufnehmenden, saugfähigen Material, und
   c) mit einer Außenlage aus einem feuchtigkeitsundurchlässigen Material, **dadurch gekennzeichnet,** daß
   d) die Außenlage (18) aus einem Vliesstoff (40) besteht,
   e) der auf einer Seite mit einer dünnen Schicht aus einem thermoplastischen, hochpolymeren Werkstoff versehen ist.

2. Hygiene-Artikel nach Anspruch 1, dadurch gekennzeichnet, daß
   f) die dem Polstermaterial (14) zugewandte Innenseite des Vliesstoffes (40) mit dem thermoplastischen, hochpolymeren Werkstoff beschichtet ist.

3. Hygiene-Artikel nach einem der Ansprüche 1 oder 2, dadurch gekennzeichnet, daß
   g) als thermoplastischer Werkstoff ein Hotmelt verwendet wird.

4. Hygiene-Artikel nach einem der Ansprüche 1 bis 3, dadurch gekennzeichnet, daß
   h) der thermoplastische Werkstoff mit einem Flächengewicht von 15 bis 30 g/m² auf den Vliesstoff (40) aufgebracht ist.

5. Hygiene-Artikel nach einem der Ansprüche 1 bis 4, dadurch gekennzeichnet, daß
   i) als thermoplastischer Werkstoff ein Hotmelt auf der Basis von Polyethylen, EVA oder ATP verwendet wird.

6. Hygiene-Artikel nach einem der Ansprüche 1 bis 5, dadurch gekennzeichnet, daß
   j) als Befestigungsvorrichtung ein Klebeband (20) vorgesehen ist, das mit der unbeschichteten Außenfläche des Vliesstoffe (40) der Außenlage (18) verbindbar ist

7. Hygiene-Artikel nach einem der Ansprüche 1 bis 6, dadurch gekennzeichnet, daß
   k) der Vliesstoff (40) der Außenlage (18) aus einem hochpolymeren Fasermaterial besteht.

8. Hygiene-Artikel nach Anspruch 7, dadurch gekennzeichnet, daß

l) der Vliesstoff (40) der Außenlage (18) aus Polypropylen-, Polyamid- oder Polyester-Fasern besteht.

9. Verfahren zur Herstellung eines Hygiene-Artikels,

a) bei dem eine Innenlage aus einem feuchtigkeitsdurchlässigen Material auf eine Feuchtigkeit aufnehmende, saugfähige Polsterlage kaschiert wird, dadurch gekennzeichnet, daß

b) eine Vliesstoff-Bahn, die auf einer Seite mit einem thermoplastischen, hochpolymeren Werkstoff beschichtet ist, als feuchtigkeitsundurchlässige Außenlage auf die Polsterlage aufkaschiert wird.

10. Verfahren nach Anspruch 9, dadurch gekennzeichnet, daß

c) eine vorbeschichtete Vliesstoff-Bahn verwendet wird.

11. Verfahren nach Anspruch 9, dadurch gekennzeichnet, daß

d) die Vliesstoff-Bahn auf einer Seite mit dem thermoplastischen, hochpolymeren Werkstoff beschichtet und unmittelbar anschließend auf die Polsterlage aufkaschiert wird.

12. Verfahren nach einem der Ansprüche 9 bis 11, dadurch gekennzeichnet, daß

e) der thermoplastische, hochpolymere Werkstoff mit einer Flächendichte im Bereich von 15 bis 30 g/m² aufgebracht wird.

13. Verfahren nach einem der Ansprüche 9 bis 12, dadurch gekennzeichnet, daß

f) als thermoplastischer, hochpolymerer Werkstoff ein Hotmelt verwendet wird.

14. Verfahren nach Anspruch 13, dadurch gekennzeichnet, daß

g) ein Hotmelt auf der Basis von Polyethylen, EVA oder ATP verwendet wird.

15. Verfahren nach einem der Ansprüche 9 bis 14, dadurch gekennzeichnet, daß

h) ein Vliesstoff aus Polypropylen-, Polyamid- oder Polyester-Fasern verwendet wird.

16. Verfahren nach einem der Ansprüche 9 bis 15, dadurch gekennzeichnet, daß

i) der mit dem Hotmelt beschichtete Vliesstoff um eine Heizwalze geführt und dann auf das Polstermaterial aufkaschiert wird.

17. Verfahren nach einem der Ansprüche 9 bis 16, dadurch gekennzeichnet, daß

j) der thermoplastische, hochpolymere Werkstoff mit einer Flächendüse aufgebracht wird.

18. Verfahren nach einem der Ansprüche 9 bis 16, dadurch gekennzeichnet, daß

k) der thermoplastische hochpolymere Werkstoff mit einer Auftragwalze aufgebracht wird.

FIG. 1

POOR QUALITY

Schmelzklebstoff

FIG. 2

Schmelzklebstoff

Fig. 3